# EUROPÄISCHE PATENTSCHRIFT

(11) **EP 0 523 395 B1**
(45) Veröffentlichungstag und Bekanntmachung des Hinweises auf die Patenterteilung: **04.10.2001**
(21) Anmeldenummer: 92110430.3
(22) Anmeldetag: 20.06.1992
(51) Int. Cl.: C07K 7/02, C07K 14/025, A61K 39/12, C12P 21/08, G01N 33/569

(54) **Seroreaktive Bereiche auf den HPV 16 Proteinen E1 und E2**
Seroreactive domains from the HPV 16 E1 and E2 proteins
Domaines séroréactives des protéines E1 et E2 d'HPV 16

(30) Priorität: 18.07.1991 DE 4123760
(43) Veröffentlichungstag der Anmeldung: 20.01.1993
(60) Teilanmeldung: 01102056.7
(73) Patentinhaber: Dade Behring Marburg GmbH, 35001 Marburg (DE)
(72) Erfinder: Müller, Martin, W-6900 Heidelberg (DE); Gissmann, Lutz, W-6908 Wiesloch (DE)

(56) Entgegenhaltungen:
- EP-A- 0 257 754
- EP-A- 0 344 940
- WO-A-91/18294
- INT. J. CANCER, Bd.46, 1990 Seiten 703 - 711 J. DILLNER 'Mapping of linear epitopes of human papillomavirus 16'

## Beschreibung

Die Erfindung betrifft seroreaktive Bereiche auf dem Protein E1 des menschlichen Papillomavirus (HPV) 16.

Weiterhin betrifft die Anmeldung einen Impfstoff, der solche Peptide enthält, die die seroreaktiven Bereiche enthalten.

Die Erfindung umfaßt ebenfalls Zusammensetzungen für diagnostische Zwecke, die Peptide mit den seroreaktiven Bereichen enthalten.

HPV 16 gehört zu den menschlichen Papillomaviren (Proc. Natl. Acad. Sci., USA 80, 3813-3815 (1983). Die Organisation des Genoms von HP 16 wurde in Virology 145, 181-185 (1985) beschrieben.

Angaben zur Struktur linearer Epitope auf verschiedenen HPV 16 ORF (E1, E2, E4, E5, E6, E7) finden sich in Int. J. Cancer 46, 703-711 (1990). Die Untersuchung des E1 ORF ergab nur eine geringe Immunreaktivität; es konnten lediglich schwache Epitope im carboxyterminalen Bereich gefunden werden.

Genomische Sequenzen des HPV können in den meisten Fällen von präinvasiven und invasiven zervikalen Tumoren nachgewiesen werden. HPV 16 ist als der in diesen Tumoren dominierende Virus Typus weltweit erkannt worden. Das HPV 16 Genom ist in über 50 % von zervikalen Tumoren nachweisbar, wobei es oft in die zelluläre DNA integriert vorliegt. Wenig ist über die Immunantwort nach Infektionen mit HPV 16 oder andere Papillomaviren bekannt.

Erste Daten: Es wurden Patienten, die an zervikalen Tumoren litten, mit gesunden Individuen im Hinblick auf die Anwesenheit von Antikörpern gegen virale Proteine verglichen. Diese viralen Proteine wurden dann als Fusionsprodukte mit unterschiedlichen prokaryotischen Peptiden an ihrem N-Terminus verbunden und dann als Antigene in Western-Blots verwendet.

Die Aufgabe der vorliegenden Erfindung ist die weitere Identifizierung von viralen Strukturen des HPV 16, die als Werkzeug in der Prophylaxe, Diagnose und Therapie von HPV 16 abhängigen Tumor-Erkrankungen des Menschen dienen können. Die Identifizierung solcher Strukturen ist eine Voraussetzung für die Entwicklung von ELISA, die es möglich machen, eine große Menge von menschlichen Seren auf Anwesenheit von HPV 16 zu testen.

Die vorliegende Erfindung umfaßt deshalb seroreaktive Bereiche des E1-Proteins von HPV 16, gekennzeichnet durch eine der folgenden Aninosäure-Sequenzen:

I.    NGWFYVEAVVEKKTGDAISDDENENDSDTGEDLVDFIVNDNDYLT

II.   NENDSDTGEDLVDFIVND

IV.   EDLVDFIVNDNDYLT

V.    EDLVDFIVNDNDYLTQAETETAHALFTAQEAKQH

VI.   NENDSDTGEDLVDFIVNDNDYLTQAETETAHALFTAQEAKQHRDAVQVLKRKYL

VII. GSPLSDIS;

Die Erfindung umfaßt weiterhin Peptide mit einem oder mehreren der oben bezeichneten seroreaktiven Bereiche, einen Impfstoff, der eines oder mehrere der oben bezeichneten Peptide enthält, eine Zusammensetzung für diagnostische Zwecke zur Identifizierung von spezifischen Antikörpern gegen HPV E1-Protein, die ebenfalls die oben bezeichnenten Peptide enthalten, sowie monoklonale Antikörper, die eine Affinität gegenüber einem oder mehreren der seroreaktiven Bereiche des E1-Proteins von HPV 16 besitzen sowie eine Zusammensetzung für diagnostische Zwecke, die diese monoklonalen Antikörper enthält.

Um eine Identifizierung von seroreaktiven Bereichen im Protein E1 von HPV durchzuführen, wurde der in Science 228, 1315-1317 (1985) beschriebene experimentelle Weg beschritten. Subgenomische, zufällig durch Ultraschallbehandlung und partielle DNAse I Behandlung generierte HPV 16 DNA Fragmente wurden in den Phagenvektor fuse1 kloniert und dort als Teil eines Phagenhüllproteins exprimiert. Seroreaktive Phagenrekombinanten wurden mit gegen E1 hergestellten Seren identifiziert, gereinigt und durch Sequenzierung des HPV 16-Anteils wurden die seroreaktiven Bereiche charakterisiert. Polyklonale Kaninchenseren wurden gegen ein HPV 16 E1 MS2 Polymerase Fusionsprotein sowie gegen den amino- und carboxyterminalen Teil von HPV 16 E2 (getrennt, ebenfalls MS2 Fusionsproteine) hergestellt.

Die filamentösen Phagen umfassen die drei Gruppen f1, fd und M13. Allen gemeinsam ist, daß Bindung und Aufnahme der Phagen über F-Pili der Bakterien erfolgt, d.h., daß nur F⁺ Stämme infiziert werden können. Der fd Wildtyp-Phage, von dem sich das verwendete Vektorsystem ableitet, bildet ca. 900 x 6 nm große Partikel, welche vor allem aus etwa 2700 Untereinheiten des Haupthüllproteins bestehen. Zusätzlich sind auf beiden Enden der Virionen jeweils 5 Moleküle der "Minor"-Hüllproteine pIII, pVI, pVII und pIX angeordnet. Das einzelsträngige, zirkuläre, beim fd Wildtyp 6408 bp große Phagengenom trägt die Information für insgesamt 10 verschiedene Proteine.

Bei den fd Abkömmlingen fuse1, fuse2 (Parmley and Smith, Gene, 7, 305-318 (1988)) und fusemm ist durch Insertion eines Teils des Tn10 Transposons ein Tetrazyklinresistenzgen im Phagengenom integriert, welches dadurch auf etwa 9,2 kbp vergrößert wurde. Dadurch verhalten sich die replikativen, DNA-doppelsträngigen Phagengenome in den Bakterien wie selektierbare Plasmide und lassen sich dementsprechend präparieren und für Klonierungen verwenden. Eine weitere Veränderung gegenüber dem Wildtyp ist eine vorhandene Leserastermutation im Gen für das "Minor"-Hüllprotein pIII in Verbindung mit einer eingeführten Restriktionsstelle zur Klonierung exprimierbarer DNA Teilstücke. Das Gen für pIII besteht aus zwei fast völlig unabhängigen Domänen (Crissmann and Smith, 1984): Einer N-terminalen Domäne, welche die Bindung der Phagen an den Bakterienzell-Rezeptor (F-Pili) vermittelt und einer C-terminalen Proteindomäne, welche für die Phagenmorphogenese verantwortlich ist. Die Leserastermutation, welche direkt hinter der Signalsequenz des Proteins liegt, führt also zur Inaktivierung des Gens und verhindert folglich auch die Bildung infektiöser Partikel. Dies ist von Bedeutung für die Vermehrung dieser Phagenmutanten als Plasmide, da die in der Morphogenese inaktivierten fd-Genome die Wirtsbakterien nicht schädigen (Smith, in: Vectors: A Survey of Molecular Cloning Vectors and Their Uses, Butterworth Publishers, Stoneham, MA 61-85, 1987).

Durch Einfügen geeigneter DNA Fragmente und Wiederherstellen der Gen III Funktionen werden infektiöse Phagenpartikel gebildet, die zusätzliche Aminosäuresequenzen auf ihren Hüllen tragen. Diese Sequenzen sind im nativen Zustand der Phagen verschiedenen Liganden, z.B. Antikörpern, zugänglich.

Das bei dieser Erfindung verwendete fd Expressionssystem beruht im wesentlichen darauf, Phagenbänke durch Einklonierung von DNA-Fremdsequenzen in das Gen III anzulegen und diese mit Hilfe von monoklonalen oder polyklonalen Seren auf seroreaktive Rekombinanten zu untersuchen. Üblicherweise erfolgt beim Herstellen dieser Expressionsbanken eine Amplifikation. Das Ausmaß dieser Vermehrung einzelner Klone ist wiederum abhängig von der Art und Größe der eingefügten DNA Sequenz. Dadurch kommt es zu einer unterschiedlichen Häufigkeit verschiedener Klone, die bis zu mehreren Zehnerpotenzen differieren kann. Aus den aufgeführten Eigenschaften lassen sich deshalb folgende zwei Merkmale der fd Expressionsbanken ableiten:
- Amplifikation der Banken, was zum wiederholten Klonieren identischer durch Immunoscreening isolierter Phagenklone führt.
- Möglichkeit der Anreicherung seroreaktiver Phagen durch Affinitätschromatographie (Säulen), da Phagen im aktiven Zustand gebunden und wieder eluiert werden können.

Die wiederholte Isolierung identischer Rekombinanter wurde umgangen, indem getrennt angelegte Banken verwendet wurden, wobei die Wahrscheinlichkeit der Klonierung eines parallel identisch hergestellten DNA Fragments bzw. des daraus abgeleiteten Phagenrekombinanten äußerst gering ist.

Bei dieser Erfindung wurden insgesamt 11 verschiedene Expressionsbanken für HPV 16 DNA in fuse1 angelegt. Die Anzahl der primären, Tetrazyklin-resistenten und Insert tragenden Rekombinanten betrug dabei zwischen 2000 und 90000 pro Bank. Da für die Klonierung stets komplette Plasmide, bestehend aus ca. 4 kb Vektoranteil und 8 kb HPV Anteil in gescherter Form verwendet wurden, reduzieren sich die HPV haltigen fd Rekombinanten um etwa 30 %. Die Expression der einklonierten Fragmente erfolgte dann, wie bereits erwähnt, als Fusionsprotein des Gen III Hüllproteins. Die Klonierungsstelle im Gen III liegt dabei direkt hinter der translatierten Signalsequenz für den Proteinexport. Um die Funktion des Gens wiederherzustellen, muß ein Insert eine definierte Größe (3n+2; n= 0, 1, 2, 3 ...) besitzen. Um eine definierte Proteinsequenz als Fusionsprotein des Gen III-Produktes zu exprimieren, muß zusätzlich sowohl der 3' als auch der 5' Übergang im richtigen Leseraster erfolgen, sowie das entsprechende Insert in der richtigen Orientierung vorliegen. Dies ist somit insgesamt nur etwa für jeden 18ten (3x3x2) HPV-DNA haltigen Rekombinanten zutreffend. Ein kleiner Teil davon wiederum wird durch im Insert vorhandene Stopp-Kodone der Translation oder durch auf Grund ihrer Faltung nicht funktionelle Proteine inaktiviert. Wegen der aufgeführten Parameter ist es schwer abzuschätzen, welche Mindestzahl an Rekombinanten nötig ist, um mit großer Wahrscheinlichkeit jeden beliebigen Teil eines HPV-Genoms als fd-Fusionsprotein in der Phagenbank zu exprimieren. Bei Papillomaviren sind etwa 10 kb des Genoms (z.T. durch überlappende offene Leseraster) für Proteine kodierend. Bei 2000 Tetrazyklin resistenten Insert tragenden Rekombinanten exprimieren etwa 100 (1/18) Klone HPV Sequenzen in geeigneter Weise. Bei durchschnittlichen HPV Fragmentgrößen von 50-150 bp beträgt die exprimierte HPV Sequenz etwa 5000-15000 bp. Tatsächlich erwiesen sich fd Banken mit etwa 2000 Rekombinanten als ausreichend repräsentativ.

Um die Spezifität des Immunoscreenings sicher zu stellen, wurden stets entweder mehrere verschiedene Rekombinante einer seroreaktiven Region, oder zumindest mehrere identische, aber unabhängig isolierte Phagenrekombinanten isoliert.

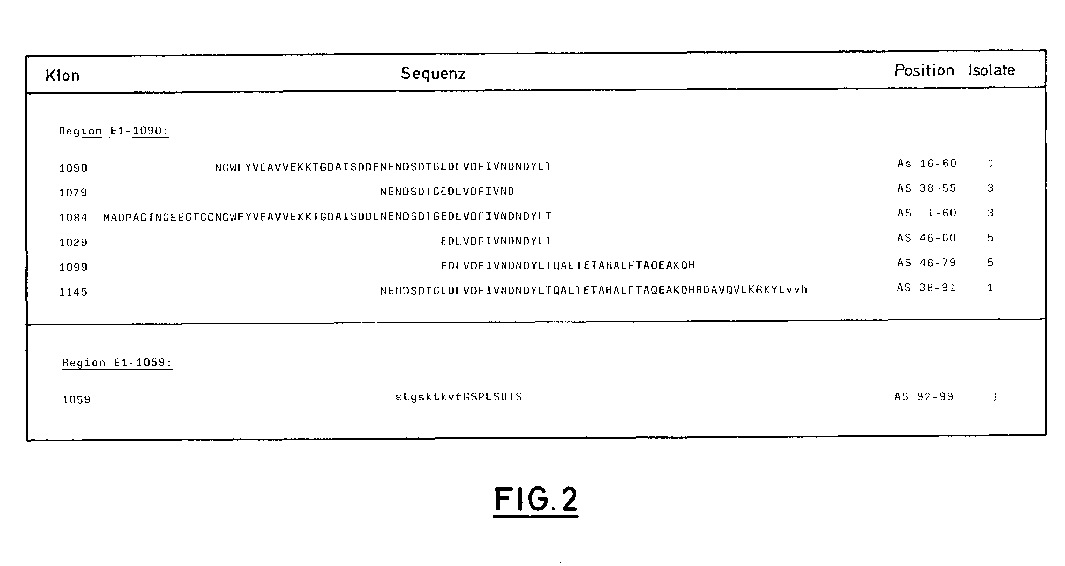

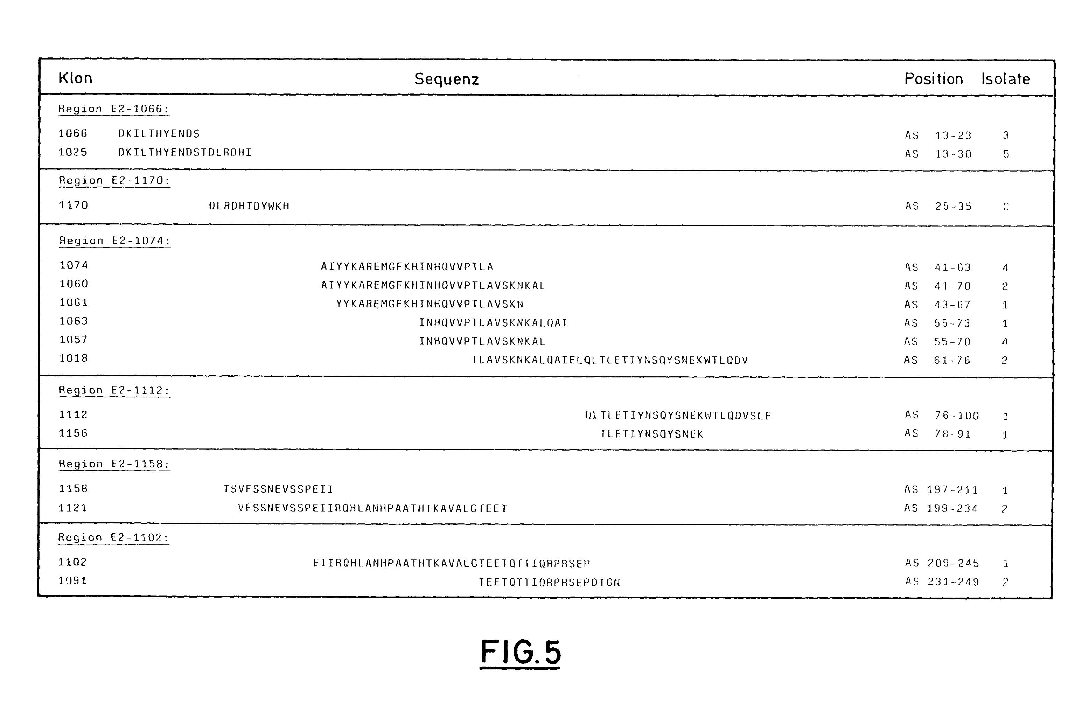

Die Aminosäure-Positionsangaben in den nachstehenden Figuren 2 und 5 beziehen sich auf die E1- und E2-Proteine und nicht auf die Positionen der offenen Leseraster. Das erste Methionin erhielt die Position 1.

In den nachfolgenden Beispielen 1, 2 und 7 wird die vorliegende Erfindung weiter ausgeführt. Bei den weiteren Beispielen 3 - 6 handelt es sich um Vergleichsbeispiele, deren Gegenstände nicht beansprucht werden.

### Beispiel 1

### Herstellen polyklonaler Antiseren gegen HPV 16 E1

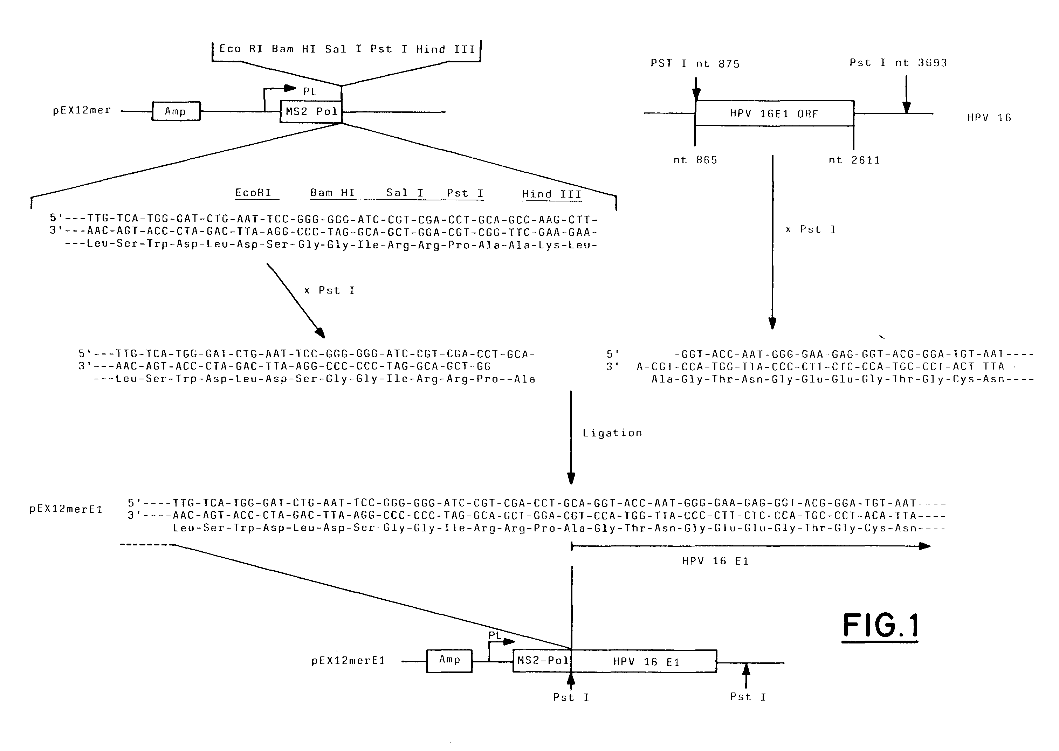

Um seroreaktive Phagenrekombinante aus der HPV 16 fd-Expressionsbank zu isolieren, wurden zunächst polyklonale Kaninchenseren gegen HPV 16 E1 MS2 Fusionsproteine hergestellt. Dazu wurde das Pst I A Fragment von HPV 16 (bp 875-3693) in die PST I Schnittstelle des Expressionsvektors pEX12mer (Seedorf et al., EMBO J. 6, 139-144, 1987) einkloniert, wodurch die Aminosäuren 5-649 des HPV 16 E1 ORF exprimiert werden (Fig. 1). Dieser Vektor ist ein Derivat des Plasmids pPLC24 (Remaut et al., Gene 15, 81-93, 1981), welcher durch Einfügen des pUC8-Polylinkers hinter den MS2 Polymerase Anteil modifiziert wurde. Das Fusionsprotein wird im pEX12mer vom Temperatur-induzierbaren Lambda-pL-Promotor transkribiert. Der N-terminale Fusionsanteil des MS2 Proteins beträgt 100 Aminosäuren.

Da das ursprüngliche HPV 16 Isolat (Seedorf et al., Virology, 145, 181-185, 1985) eine Leserastermutation im Bereich des E1 offenen Leserasters (Nukleotidposition 1138) aufweist, wurde auf ein HPV 16 Isolat aus einem Zervix-Karzinom mit einem vollständigen E1 ORF zurückgegriffen. Auf Grund der gewählten Restriktionsschnitte wird das HPV 16 E1 offene Leseraster (bp 865-2811) somit bis auf drei N-terminale Aminosäuren vollständig exprimiert.

Die Klonierungen und Plasmidanalysen wurden zunächst unter Verwendung des E. coli Stammes W6 durchgeführt, welcher den Repressor für den Lambda Promotor konstitutiv exprimiert. Dadurch wurde die Expression der Fusionsproteine verhindert, um eine Gegenselektion nach den Transformationen zu verhindern. Nach Überprüfung der Klonierung durch Restriktionsanalyse, sowie Southern-Blot Hybridisierung mit radioaktiv markierter HPV 16 DNA (Pst I A Fragment) wurde die Plasmid-DNA des Konstruktes zur Transformation in E. coli N6045 verwendet. Dieser Stamm ist aufgrund seines temperatursensitiven Repressors des Lambda Promotors zur Expression der MS2 Fusionsproteine in der Lage.

Im Western-Blot konnte dann mit Hilfe eines gegen den MS2-Anteil des Fusionsproteins gerichteten monoklonalen Antikörpers durch Vergleich von Extrakten aus induzierten und nicht-induzierten Bakterien die Größe und die Expressionsrate des Fusionsproteins überprüft werden. Da die Bande des MS2 E1 Fusionsproteins der erwarteten Größe von ca. 90 kD entsprach, wurde keine Überprüfung der Klonierungsübergänge durch Sequenzierung vorgenommen. In den beiden anderen Leserastern des HPV 16 Pst I A Fragmentes ist die Expression größerer Proteine wegen vorhandener Stoppkodone der Translation nicht möglich. Zudem waren beide Pst I Schnittstellen der Vektor-Insert Übergänge erhalten geblieben. Die korrekte Expression des E1 offenen Leserasters wurde durch die Ergebnisse des Immunoscreenings der HPV 16 fd-Expressionsbanken, die im folgenden Kapitel beschrieben wird, bestätigt.

Das MS2-E1 Fusionsprotein wurde dann aus induzierten E. coli Kulturen durch differentielle Extraktion und durch Elektroelution aus SDS-Polyacrylamid Gelen aufgereinigt und dann zur Immunisierung von zwei Kaninchen verwendet.

### Beispiel 2

### Identifizierung seroreaktiver Bereiche auf dem HPV 16 E1 Protein

Mit Hilfe beider hergestellten polyklonalen Kaninchenseren gegen HPV 16 E1 wurden fünf verschiedene HPV 16 fd-Expressionsbanken auf reaktive Rekombinanten untersucht. Dabei konnten insgesamt mindestens zwei unterschiedliche, durch nicht überlappende Phagenklone repräsentierte Antikörperbindungsstellen identifiziert werden. Insgesamt wurden 19 unabhängige Phagenklone isoliert, die sieben unterschiedliche Klassen von HPV 16 Inserts enthalten (Fig. 2). Sechs Klassen haben einen gemeinsamen überlappenden Bereich, der für das HPV 16 E1 spezifische Peptid EDLVDFIVND kodiert. Das zweite identifizierte Epitop auf dem E1 Protein wird durch einen rekombinanten Phagen (Klon 1059) repräsentiert, welcher für das E1 Peptid GSPLSDIS kodierend ist.

Das ursprüngliche HPV 16 Isolat besitzt eine Leserastermutation im E1 offenen Leseraster (Nukleotidposition 1138). Die DNA dieses HPV 16 Isolats wurde verwendet, um die fd-Expressionsbanken herzustellen. Zwei der isolierten, seroreaktiven fd-Rekombinanten enthalten diesen Bereich und weisen dehalb auch die Leserastermutation auf. Bei Klon 1145 führt dies zu einem Leserasterwechsel und drei HPV 16-E2 unspezifische Aminosäuren (...ValValHis) werden dadurch C-terminal angehängt. Klon 1059 startet im falschen Raster und wird durch die Leserastermutation des verwendeten HPV 16 Isolats in das richtige HPV 16 E1 Leseraster überführt. Der Klon kodiert für das Peptid STGSKTKVFGSPLKSDIS von dem nur die C-terminalen Aminosäuren ...GSPLSDIS aus dem eigentlichen HPV 16 E1 Protein entstammen und das Epitop bilden müssen.

Beide Klone, die die Leserastermutation enthalten, besitzen die richtige Insertgröße (3n+2 Basenpaare), um das Leseraster des Gen III des Phagenvektors wiederherzustellen.

### Beispiel 3

### Herstellen polyklonaler Antiseren gegen HPV 16 E2

Wie im Falle des HPV 16 E1 offenen Leserasters standen auch für das HPV 16 E2 Protein keine geeigneten Antiseren zur Verfügung. Aus diesem Grund wurde das HPV 16 E2 offene Leseraster (Nukleotidposition 2756-3850; AS 1-365), wie schon für das E1 Protein beschrieben, in dem Vektor pEX12mer exprimiert.

Zunächst wurde das HPV 16 DNA Fragment über die Hinf I Schnittstelle bei Position 2761 und in den pEX12mer Vektor einkloniert. Ausgegangen wurde dabei von einem bereits subklonierten HPV 16 Fragment (bp 2367-4467). Dieses Fragment wurde über die zusätzlich eingefügten, nicht HPV 16 spezifischen Restriktionsstellen Xba I (5' Ende) und Bam HI (3' Ende) wieder aus dem Vektor ausgeschnitten und präpariert. Dieses 2,1 kb große DNA Fragment (Xba I/Bam HI) wurde dann partial mit Hinf I geschnitten. Dabei entsteht unter anderem ein 1700 bp großes Fragment zwischen der 3'terminalen Bam HI Schnittstelle und der Hinf I Stelle bei bp 2761. In diesem Fragment ist die interne Hinf I Schnittstelle (bp 3539) ungespalten und das HPV 16 E2 ORF bis auf drei aminoterminale Aminosäuren vollständig enthalten. Nach Präparation wurde das Hinf/Bam Fragment in den mit Bam HI gespaltenen pEX12mer Expressionsvektor einkloniert. Dabei entstehen über die kompatiblen Bam HI Stellen lineare Produkte aus Vektor und Insert. Die freien Enden dieser Produkte wurden mit Klenow Polymerase aufgefüllt und dann durch Ligation geschlossen. Dabei entsteht ein MS2-E2 Übergang an den aufgefüllten Schnittstellen Bam HI (Vektor) bzw. Hinf I (E2 Insert) unter Verlust der beiden Restriktionsstellen. Mit Hilfe von Eco RI/Bam HI Doppelrestriktionsspaltungen konnten Rekombinante identifiziert werden, die das HPV 16 E2 Fragment in richtiger Orientierung trugen.

Nach Transformation in den E. coli Expressionsstamm 6045 konnte bei Verwendung eines gegen die MS2-Polymerase gerichteten monoklonalen Antikörpers keinerlei Produktion des MS2-Fusionsproteins festgestellt werden. Um eine Leserasterverschiebung am MS2-E2 Übergang auszuschließen, wurde die Plasmid DNA von insgesamt 16 unterschiedlichen MS2-E2 Rekombinanten im Southern-Blot mit einem aus dem korrekten Bam HI/Hinf I Übergang abgeleiteten Oligonukleotid hybridisiert. Da bei 15 Klonen ein eindeutiges Hybridisierungssignal zu erkennen war, wurde davon ausgegangen, daß die Klonierung im richtigen Leseraster erfolgt war und die Expression des kompletten E2 ORF in pEX Vektoren nicht möglich ist. Ersatzweise wurde das HPV 16 E2 Protein dann in zwei Hälften im pEX12mer Vektor exprimiert.

### Beispiel 4

### Expression des aminoterminalen Bereichs von HPV 16 E2

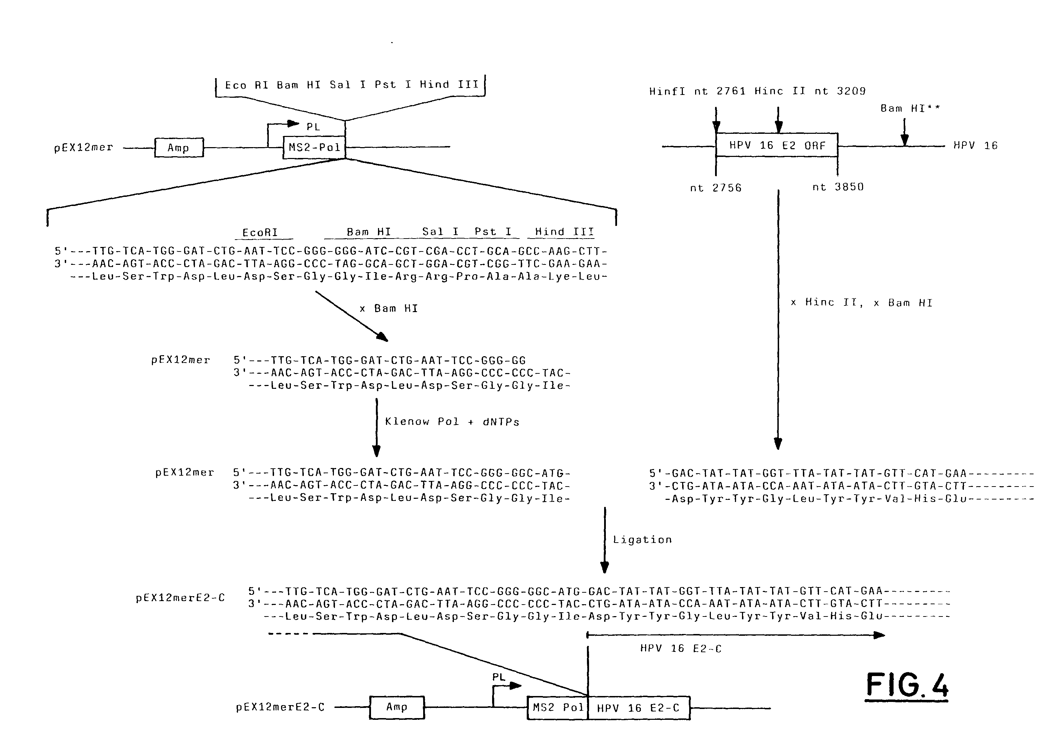

Der aminoterminale Bereich des E2 offenen Leserasters zwischen Nukleotidposition 2761 und 3209 wurde in den pEX12mer Vektor kloniert und exprimiert. Da das E2 offene Leseraster bei Nukleotidposition 2756 beginnt, fehlen im MS2-E2 Fusionsprotein die ersten beiden Aminosäuren (Met-Glu) des E2 Proteins (Fig. 4).

Plasmid DNA bestehend aus pEX12mer und HPV 16 E2, welche aus oben beschriebener Klonierung hervorgegangen waren, wurde durch Herausspalten eines Hinc II (HPV 16 bp 3209)/ Bam HI Fragments und Religation (glatt/stumpf aus Hinc II und Bam HI) carboxyterminal verkürzt. Dadurch wird der N-terminale Teil von HPV 16 E2 zwischen Nukleotidposition 2761 (Hinf I) und 3209 (Hinc II) exprimiert. Im Western-Blot konnte in induzierten Bakterien mit einem anti-MS2 monoklonalen Antikörper ein Fusionsprotein von ca. 30 kD Größe nachgewiesen werden.

Das Fusionsprotein wurde durch differentielle Extraktion der induzierten Bakterienlysate sowie durch Elektroelution der Proteinbande aus Coomassie Blau gefärbten SDS Polyacrylamid Gelen gereinigt und zur Immunisierung von Kaninchen verwendet.

### Beispiel 5

### Expression des carboxyterminalen Bereichs von HPV 16 E2

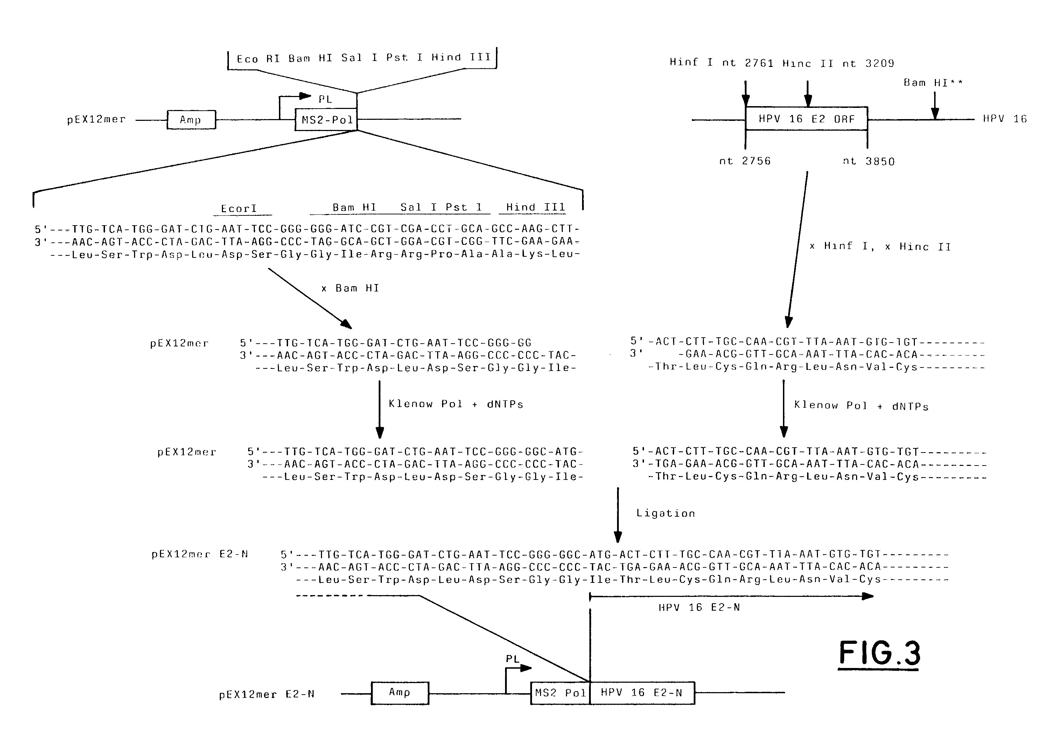

Es wurde der C-terminale Bereich des HPV 16 E2 offenen Leserasters zwischen Nukleotidposition 3209 und 3850 im pEX12mer Vektor exprimiert (Fig. 3). Der Bereich schließt sich also direkt an den oben beschriebenen exprimierten aminoterminalen Teil des HPV16 E2 offenen Leserasters an.

Dazu wurde auf das oben beschriebene Xba/Bam Fragment, das das gesamte HPV 16 E2 Leseraster enthält, zurückgegriffen. Nach Restriktionsspaltung wurde ein Hinc II/Bam HI Fragment (Nukleotidposition 3209-4467) isoliert, welches die carboxyterminale Hälfte von HPV 16 E2 enthält. Dieses Fragment wurde in die Bam HI Schnittstelle des pEX12mer Expressionsvektors eingesetzt (5'Bam HI/Hinc II--Bam HI/ Bam HI 3'). Mit Hilfe des anti MS2 monoklonalen Antikörpers konnte in Extrakten induzierter Bakterien ein Fusionsprotein von ca. 30 kD identifiziert werden, welches mittels differentieller Extraktion und Elektroelution aus SDS Polyacrylamid Gelen gereinigt und zur Immunisierung von Kaninchen verwendet wurde.

### Beispiel 6

### Identifizierung seroreaktiver Bereiche auf dem HPV 16 E2 Protein

Für das Immunoscreening der fd-HPV 16 Expressionsbanken standen insgesamt vier verschiedene anti HPV 16 E2 Antiseren zur Verfügung: Jeweils zwei Seren gegen den aminoterminalen Teil (bp 2761-3209; AS 3-152) und zwei gegen den carboxyterminalen Teil des E2 (bp 3209-3850; AS 153-365) offenen Leserasters. Mit Hilfe dieser Seren wurden fünf verschiedene Expressionsbanken auf seroreaktive Rekombinanten untersucht. Dabei wurden insgesamt 32 Klone isoliert, wovon 26 aminoterminale Sequenzen des E2 Proteins enthalten. Diese 26 Klone bilden insgesamt 11 verschiedene Klassen, die vier verschiedene, nicht überlappende Regionen repräsentieren (Fig. 5).

Alle Epitope sind in einem eng Benachbarten, 88 Aminosäuren umfassenden Bereich des Aminoterminus des E2 offenen Leserasters angeordnet, der zwischen Nukleotidposition 2792 (AspLysIle...) und 3055 (...SerLeuGlu) liegt.

Im carboxyterminalen Bereich konnten mindestens zwei unabhängige, nicht überlappende Epitope lokalisiert werden (TSVFSSNEVSSPEII und TEETQTTIQRPRISEPDTGN, Fig. 5). Diese werden von insgesamt vier Klassen von Rekombinanten bei sechs unabhängigen Isolaten repräsentiert. Der Bereich des E2 offenen Leserasters, der von den Klonen abgedeckt wird, liegt zwischen der Nukleotidposition 3343 (ThrSerVal...) und 3502 (...ThrGlyAsn) und umfaßt 52 Aminosäuren.

Fünf Klassen von Rekombinanten (12 Isolate) überstreichen die Nukleotidposition 2926. Alle Klone weisen hier eine Punktmutation (A -> G Transition) auf, die jedoch nicht zu einer Änderung der entsprechenden Aminosäure (Glutamin) führt.

### Beispiel 7

### Immunoscreening von fd-Phagenexpressionsbänken

### 1. Phagenaffinitätsanreicherung mit Protein A Sepharose Säulen

Im verwendeten fd-Phagenexpressionssystem wurden die hergestellten Phagenbänke unvermeidlich bei der Klonierung einer Amplifikation unterworfen. Das Ausmaß dieser Vermehrung der ursprünglichen Klone ist wiederum in starkem Maße von der Beschaffenheit der einzelnen Rekombinanten, etwa durch unterschiedliche Insertgrößen oder Konformation der Hüllproteine, Hemmung der physiologischen Prozesse in den infizierten Bakterien und vieles andere beeinflußt und es war deshalb nicht zu erwarten, daß eine einheitliche Amplifikation aller Phagen erfolgt. Um unterrepräsentierte Phagenrekombinante oder Klone aus großen Bibliotheken zu isolieren, wurden Anreicherungen seroreaktiver Phagenrekombinanten durchgeführt. Dazu wurde Gebrauch von dem Umstand gemacht, daß die jeweils exprimierten Fremdsequenzen als Teil eines fd-Gen III Fusionsproteins auf der Hülle nativer Phagenpartikel erscheinen. Große Phagenmengen (10⁹-10¹² Partikel) wurden dazu über Protein-A-Antikörper Säulen gebunden und wieder eluiert.

Dazu wurde zunächst Protein A Sepharose mit PBS 30 min lang gequollen und mit PBS gewaschen. Anschließend wurde die Protein A Sepharose mit etwa 1 bis 2 ml geeigneter polyklonaler Seren (Kaninchen oder Mensch) oder mit entsprechenden, Protein A bindenden monoklonalen Antikörpern, in Eppendorf-Reaktionsgefäßen auf dem Drehschüttler für 1 bis 2 Tage bei 4 °C inkubiert. Anschließend wurde die Protein A Sepharose 10 mal gewaschen, indem die Sepharose abwechselnd in 10 ml PBS resuspendiert und durch Zentrifugieren (2 min, 6000 upm) wieder pelletiert wurde. Die gebildeten Protein-A-Sepharose-IgG Komplexe wurden dann mit einer entsprechenden Phagenmenge wie oben inkubiert. Darauf wurde wie zuvor mehrmals mit PBS gewaschen, die Sepharose in eine mit einer Glaskugel verschlossene Pasteurpipette gefüllt und mit mehreren Litern (2-15 l) im Durchfluß gewaschen. Nach Herausnahme des Säulenmaterials wurde dieses für 15 min in gleichem Volumen Elutionspuffer (1 mg/ml BSA, 0,1 M HCl, mit Glycin auf pH 2,2 eingestellt) inkubiert. Anschließend wurde kurz zentrifugiert und der Überstand, der nun freie Phagen und Antikörper enthält, mit 1/5 Volumen Tris Base (0,5 M) neutralisiert. Antikörper, welche das rekombinante Gen erkennen, sind in der Lage, die Phagenbindung an die Bakterienzellen und damit den Infektionszyklus zu hemmen. Deshalb wurden die Phagen in 100-200 *µ*l Aliquots der Eluate sofort nach der Neutralisation zu exponentiell wachsenden E. coli K91 gegeben und auf Vollmediumplatten ausplattiert. Es zeigte sich im Verlauf der Arbeit, daß Replikafilter von diesen Phagenplattierungen wohl auf Grund der Beimischungen im Eluat nicht für das Immunoblotting geeignet waren. Deshalb wurden die erhaltenen Plaques erneut mit Vollmedium von den Platten gespült und später aus den so erhaltenen, erneut amplifizierten Phagensuspensionen ausplattiert und das Immunoblotting auf Minimalagarplatten durchgeführt.

### 2. Phagen-Plattierungen und Herstellen von Nitrocellulose Replikafiltern für das Immunoblotting

Alle fd-Phagenderivate wurden auf einem Rasen ausplattiert, der von E. coli K91 (Lyons and Zinder, Virology, 49, 45-60, 1972) gebildet wurde. Dieser Stamm zeichnet sich durch eine hohe Zahl von F-Pili aus (5 pro Zelle, gegenüber etwa 0,5 pro Zelle bei den meisten F⁺-Stämmen), die für die Aufnahme der filamentösen Phagen verantwortlich sind. Dies ist bei dem in dieser Arbeit verwendeten fd-Expressionssystem besonders wichtig, da die rekombinanten fuse-Phagen durch die Aufnahme eines Teils des Tn10 Transposons (Tetracyclin-Resistenz) ein gegenüber dem Wildtyp deutlich vergrößertes Genom besitzen und aus diesem Grund besonders kleine Plaques bilden.

Zum Ausplattieren der Phagen wird eine K91 Übernachtkultur 1:100 in Vollmedium (2x YT) verdünnt und 3 bis 4 h bei 37 °C inkubiert. Nach Erreichen einer Dichte von E₆₀₀ = 0,8-1,2 wurden 200 *µ*l der Bakterien mit einer entsprechenden Menge an Phagen zusammen mit 3,5 ml Agarose (0,6 % Agarose, 10 mM MgSO₄, 50 °C) auf vorgewärmte Bakterienplatten ausplattiert. Grundsätzlich wurden für sämtliche Plattierungen, von denen Nitrocellulosereplika für das "Immunoscreening" angelegt werden sollten, Minimalagarplatten benutzt. Ausplattierungen zur Phagentiterbestimmung oder für die DNA Hybridisierung wurden auf Vollmediumplatten durchgeführt.

Verwendung von Vollmediumplatten für das Immunoblotting führte stets zu sehr hoher unspezifischer Reaktivität der Filter mit den verwendeten Seren.

Die Platten wurden über Nacht bei 37 °C inkubiert. Nach ca. 16 Stunden wurde ein Nitrocellulosefilter für 10-15 min aufgelegt, mit vier asymmetrischen Einstichen einer Kanüle markiert und mit Hilfe einer Flachpinzette wieder abgenommen. Nach Beschriftung der Filter wurden diese umgekehrt auf eine frische Minimalagarplatte gelegt und für 5-6 Stunden bei 37 °C weiterinkubiert. Dadurch wurde die Menge an Phagenpartikel (-Proteinen) auf dem Filter erhöht, da durch das Reinkubieren ein weiteres Wachstum der an die Filter gebundenen Bakterien und Phagen über die von der Platte diffundierenden Nährstoffe ermöglicht wird. Anschließend wurden die Filter abgenommen und 30-60 min in 10 % Milch (Magermilchpulver in PBS) abgesättigt. Über Nacht wurden die Filter dann mit geeigneten Verdünnungen entsprechender Seren in 5 % Milch bei 4 °C inkubiert.

### 3. Immunofärbung von Replikafiltern und Klonierung von reaktiven Rekombinanten

Nach Abnahme der Replikafilter, 60 min Blocken in 10 % Milch (in PBS) und über-Nacht-Inkubation mit Antiseren, wurden die Nitrocellulosefilter 30 min in PBS, 0,05 % Tween 20 (5 Waschpufferwechsel) gewaschen. Danach wurden die Filter mit 1:1000 Verdünnungen entsprechender zweiter Antikörper (Peroxidase gekoppelte Ziege anti Human, anti Kaninchen oder anti Maus) in 5 % Milch 2 h bei RT inkubiert. Darauf folgte erneutes Waschen (s.o.) und Inkubation in folgendem Färbeansatz:

| | |
|---|---|
| 40 mg | Diaminobenzidin |
| 30 µl | 30 % H₂O₂ |
| 1,5 ml | 1 % NiSO₄ in 50 ml PBS |

Nach ausreichender Farbentwicklung wurden die Filter aus der Lösung genommen, 30 min gewässert und anschließend auf 3MM Papier getrocknet.

Einstichlöcher und Signale der Filter wurden dann auf eine Folie oder den Deckel einer Bakterienschale durchgepaust. Damit war es möglich, einem Signal eine Position, oder bei ausreichend großer Phagenverdünnung (Runde 2 oder höher) einen Plaque zuzuordnen. Mit Hilfe eines sterilen Zahnstochers wurde die Stelle oder der Plaque leicht eingestochen und der Zahnstocher 10-15 min in 500 l Vollmedium gesteckt. Diese Phagensuspension enthielt dann im allgemeinen etwa 10⁶-10⁷ infektiöse Partikel, was etwa 0,1-1 % der Phagen eines Plaques ausmacht. Die Phagensuspensionen wurden dann noch für 15-20 min bei 65 °C inkubiert, um mit übertragene Bakterien abzutöten und dann bei 4 °C aufbewahrt.

### Beschreibung der Figuren

### Fig. 1

Klonierung des E1 offenen Leserasters in den Expressionsvektor pEX12mer.

### Fig. 2

Seroreaktive Regionen auf dem HPV 16 E1 Protein. Kleinbuchstaben geben die Aminosäuren der Klone 1145 und 1059 wieder, die aufgrund des Leserasterwechsels des zur Klonierung der fd-Banken verwendeten HPV 16 Isolates nicht dem HPV 16 E1 Protein entstammen (siehe Text). Die Klone 1090, 1079, 1084, 1029, 1099 und 1145 besitzen eine gemeinsame Region von 10 Aminosäuren (EDLVDFIVND) die möglicherweise ein gemeinsames Epitop der Klone darstellt, obwohl weitere Antikörperbindungsstellen auf diesen Klonen nicht ausgeschlossen werden können. Klon 1059 besitzt aufgrund des Leserasterwechsels keine gemeinsamen Aminosäuresequenzen mit den anderen Klonen, obwohl das Insert dieses Klones mit dem Insert von Klon 1145 überlappt. Die Positionsangaben beziehen sich auf das HPV 16 E1 offene Leseraster. Die Aminosäuren der Klone 1145 und 1059, die nicht aus E1 entstammen, sind dabei nicht berücksichtigt.

### Fig. 3

Klonierung der carboxyterminalen Hälfte des HPV 16 E2 Proteins in den Expressionsvektor pEX12mer.

### Fig. 4

Klonierung der aminoterminalen Hälfte des HPV 16 E2 Proteins in den Expressionsvektor pEX12mer.

### Fig. 5

Seroreaktive Regionen auf dem HPV 16 E2 Protein.
Die Regionen (E2-1066, -1170, -1074, -1112) auf der carboxyterminalen Hälfte des HPV 16 E2 sind alle in einem 88 Aminosäuren langen Bereich (zwischen AS 13 und 100) angeordnet und überlappen teilweise. Ebenso befinden sich die carboxyterminalen Regionen eng benachbart (zwischen AS 197 und 249). Die Regionen aus beiden Bereichen sind jeweils etwa proportional zu ihrer Lage auf dem E2 Protein angeordnet.

SEQUENCE LISTING

Seq. Nr. I
NGWFYVEAVVEKKTGDAISDDENENDSDTGEDLVDFIVNDNDYLT
Human Papillomavirus 16 E1 Protein
Peptid

Seq. Nr. II
NENDSDTGEDLVDFIVND
Human Papillomavirus 16 E1 Protein
Peptid

Seq. Nr. IV
EDLVDFIVNDNDYLT
Human Papillomavirus 16 E1 Protein
Peptid

Seq. Nr. V
EDLVDFIVNDNDYLTQAETETAHALFTAQEAKQH
Human Papillomavirus 16 E1 Protein
Peptid

Seq. Nr. VI
NENDSDTGEDLVDFIVNDNDYLTQAETETAHALFTAQEAKQHRDAVQVLKRKYL
Human Papillomavirus 16 E1 Protein
Peptid

Seq. Nr. VII
GSPLSDIS
Human Papillomavirus 16 E1 Protein
Peptid

## Patentansprüche (Patentansprüche für folgende(n) Vertragsstaat(en): AT,BE,CH,LI,DE,DK,FR,GB,IT,LU,NL,PT,SE)

1. Seroreaktive Bereiche auf dem E1-Protein des menschlichen Papillomavirus (HPV) 16, **gekennzeichnet durch** die folgenden Aminosäuresequenzen:

I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS

2. Peptide, **dadurch gekennzeichnet, daß** sie einen oder mehrere der seroreaktiven Bereiche nach Anspruch 1 enthalten.

3. Impfstoff, **dadurch gekennzeichnet, daß** er eines oder mehrere der Peptide nach Anspruch 2 enthält.

4. Zusammensetzung für diagnostische Zwecke zur Identifizierung von spezifischen Antikörpern gegen HPV 16 E1 Protein, **dadurch gekennzeichnet, daß** sie Peptide nach Anspruch 2 enthält.

5. Monoklonaler Antikörper, **dadurch gekennzeichnet, daß** er eine Affinität zu den seroreaktiven Bereichen gemäß Anspruch 1 besitzt.

6. Zusammensetzung für diagnostische Zwecke, **dadurch gekennzeichnet, daß** sie einen monoklonalen Antikörper nach Anspruch 5 enthält.

7. Zusammensetzung für diagnostische Zwecke nach Anspruch 6 zur Identifizierung von HPV 16 spezifischem E1 Protein.

8. Verwendung von Peptiden nach Anspruch 2 zur Herstellung von Impfstoff oder für Zusammensetzungen für diagnostische Zwecke.

## Patentansprüche (Patentansprüche für folgende(n) Vertragsstaat(en): ES)

1. Verfahren zur Herstellung von seroreaktiven Peptiden auf dem E1-Protein des menschlichen Papillomavirus (HPV) 16 enthaltend die folgenden Aminosäuresequenzen: 
I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS
 **dadurch gekennzeichnet, daß** die genannten Sequenzen peptidchemisch oder gentechnologisch hergestellt werden.

2. Verfahren zur Herstellung von seroreaktiven Peptiden, **dadurch gekennzeichnet, daß** mehrere der Peptide hergestellt nach Anspruch 1, verbunden werden.

3. Verfahren zur Herstellung eines Impfstoffes, **dadurch gekennzeichnet, daß** eines oder mehrere der Peptide, hergestellt nach mindestens einem der Ansprüche 1-2, verwendet werden.

4. Verfahren zur Herstellung einer Zusammensetzung für diagnostische Zwecke zur Identifizierung von spezifischen Antikörpern gegen HPV 16 E1 Protein, **dadurch gekennzeichnet, daß** sie eines oder mehrere Peptide, hergestellt nach mindestens einem der Ansprüche 1-2, verwendet werden.

5. Verfahren zur Herstellung eines monoklonalen Antikörpers, **dadurch gekennzeichnet, daß** ein Peptid, hergestellt nach mindestens einem der Ansprüche 1-2, verwendet wird.

6. Verfahren zur Herstellung einer Zusammensetzung für diagnostische Zwecke, **dadurch gekennzeichnet, daß** ein monoklonaler Antikörper, hergestellt nach Anspruch 5, verwendet wird.

7. Verwendung der nach Anspruch 6 hergestellten Zusammensetzung zur Identifizierung von HPV 16 spezifischem E1 Protein.

8. Verwendung eines nach Anspruch 1 oder 2 hergestellten Peptids zur Identifizierung von spezifischen Antikörpern gegen HPV 16 E1 Protein.

9. Verwendung eines nach Anspruch 1 oder 2 hergestellten Peptids zur Vakzinierung.

## Claims (Claims for the following Contracting State(s): AT,BE,CH,LI,DE,DK,FR,GB,IT,LU,NL,PT,SE)

1. Seroreactive regions on the E1 protein of human papillomavirus (HPV) 16, with the following amino-acid sequences:

I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS

2. A peptide which contains one or more of the seroreactive regions as claimed in claim 1.

3. A vaccine which contains one or more of the peptides as claimed in claim 2.

4. A composition for diagnostic purposes for identifying specific antibodies against HPV 16 E1 protein, which contains peptides as claimed in claim 2.

5. A monoclonal antibody which has affinity for the seroreactive regions of claim 1.

6. A composition for diagnostic purposes, which contains a monoclonal antibody as claimed in claim 5.

7. A composition for diagnostic purposes as claimed in claim 6 for identifying HPV 16 specific E1 protein.

8. The use of peptides as claimed in claim 2 for producing vaccine or for compositions for diagnostic purposes.

## Claims (Claims for the following Contracting State(s): ES)

1. A process for preparing seroreactive peptides on the E1 protein of human papillomavirus (HPV) 16 comprising the following amino acid sequences: 
I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS
 wherein said sequences are prepared by methods of peptide chemistry or genetic manipulation.

2. A process for preparing seroreactive peptides, which comprises linking a plurality of peptides prepared as claimed in claim 1.

3. A process for producing a vaccine, which comprises using one or more of the peptides prepared as claimed in at least one of claims 1-2.

4. A process for producing a composition for diagnostic purposes to identify specific antibodies against HPV 16 E1 protein, wherein one or more peptides prepared as claimed in at least one of claims 1-2 are used.

5. A process for preparing a monoclonal antibody, which comprises using a peptide prepared as claimed in at least one of claims 1-2.

6. A process for producing a composition for diagnostic purposes, which comprises using a monoclonal antibody prepared as claimed in claim 5.

7. The use of the composition produced as claimed in claim 6 for identifying HPV 16-specific E1 protein.

8. The use of a peptide prepared as claimed in claim 1 or 2 for identifying specific antibodies against HPV 16 E1 protein.

9. The use of a peptide prepared as claimed in claim 1 or 2 for vaccination.

## Revendications (Revendications pour l'(les) Etat(s) contractant(s) suivant(s): AT,BE,CH,LI,DE,DK,FR,GB,IT,LU,NL,PT,SE)

1. Domaines séroréactifs sur la protéine E1 du papillomavirus humain (HPV) 16, **caractérisés par** les séquences d'aminoacides suivantes :

I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS

2. Peptides, **caractérisés en ce qu'**ils comportent un ou plusieurs des domaines séroréactifs selon la revendication 1.

3. Vaccin, **caractérisé en ce qu'**il contient un ou plusieurs des peptides selon la revendication 2.

4. Composition à des fins de diagnostic, pour l'identification d'anticorps dirigés contre la protéine E1 de HPV 16, **caractérisée en ce qu'**elle contient des peptides selon la revendication 2.

5. Anticorps monoclonal, **caractérisé en ce qu'**il possède une affinité pour les domaines séroréactifs selon la revendication 1.

6. Composition à des fins de diagnostic, **caractérisée en ce qu'**elle contient un anticorps monoclonal selon la revendication 5.

7. Composition à des fins de diagnostic selon la revendication 6, pour l'identification de la protéine E1 de HPV 16.

8. Utilisation de peptides selon la revendication 2, pour la fabrication d'un vaccin ou pour des compositions à des fins de diagnostic.

## Revendications (Revendications pour l'(les) Etat(s) contractant(s) suivant(s): ES)

1. Procédé pour la production de peptides séroréactifs sur la protéine E1 du papillomavirus humain (HPV) 16, contenant les séquences d'aminoacides suivantes : 
I.	NGWFYVEAVVEKKTGDAISDDENENDSDTGEDLVDFIVNDNDYLT
II.	NENDSDTGEDLVDFIVND
IV.	EDLVDFIVNDNDYLT
V.	EDLVDFIVNDNDYLTQAETETAHALFTAQEAKQH
VI.	NENDSDTGEDLVDFIVNDNDYLTQAETETAHALFTAQEAKQHRDAVQ
	VLKRKYL
VII.	GSPLSDIS
 **caractérisé en ce que** lesdites séquences sont produites par voie chimique de la chimie des peptides ou par génie génétique.

2. Procédé pour la production de peptides séroréactifs, **caractérisé en ce que** plusieurs des peptides produits selon la revendication 1 sont raccordés.

3. Procédé pour la fabrication d'un vaccin, **caractérisé en ce qu'**on utilise un ou plusieurs des peptides produits selon au moins une des revendications 1-2.

4. Procédé pour la préparation d'une composition à des fins de diagnostic pour l'identification d'anticorps spécifiques contre la protéine E1 de HPV 16, **caractérisé en ce qu'**on utilise un ou plusieurs peptides produits selon au moins une des revendications 1-2.

5. Procédé pour la production d'un anticorps monoclonal, **caractérisé en ce qu'**on utilise un peptide produit selon au moins une des revendications 1-2.

6. Procédé pour la préparation d'une composition à des fins de diagnostic, **caractérisé en ce qu'**on utilise un anticorps monoclonal produit selon la revendication 5.

7. Utilisation de la composition préparée selon la revendication 6, pour l'identification de la protéine E1 spécifique de HPV 16.

8. Utilisation d'un peptide produit selon la revendication 1 ou 2, pour l'identification d'anticorps spécifiques contre la protéine E1 de HPV 16.

9. Utilisation d'un peptide produit selon la revendication 1 ou 2, pour la vaccination.
